# EUROPÄISCHE PATENTSCHRIFT

(11) **EP 0 508 353 B1**
(45) Veröffentlichungstag und Bekanntmachung des Hinweises auf die Patenterteilung: **26.06.1996**
(21) Anmeldenummer: 92105934.1
(22) Anmeldetag: 06.04.1992
(51) Int. Cl.: C07D 239/46

(54) **Verfahren zur Herstellung von 4-Amino-2-chlor-5-cyan-6-(methylthio)pyrimidin**
Process for the preparation of 4-amino-2-chloro-5-cyano-6-(methylthio)pyrimidine
Procédé de préparation de 4-amino-2-chloro-5-cyano-6-(méthylthio)pyrimidine

(30) Priorität: 11.04.1991 CH 1085/91
(43) Veröffentlichungstag der Anmeldung: 14.10.1992
(73) Patentinhaber: LONZA AG, CH-3945 Gampel/Wallis (CH)
(72) Erfinder: Ryan, Gary, Dr., Visp (Kanton Wallis) (CH); Mettler, Hans Peter, Dr., Flemington, NJ 08822 (US); Previdoli, Felix, Dr., Brig (Kanton Wallis) (CH)
(74) Vertreter: Weinhold, Peter, Dr.

(56) Entgegenhaltungen:
- JOURNAL OF THE CHEMICAL SOCIETY Bd. 9, 1974, LONDON Seite 350; H. KRISTINSSON: 'SYNTHESIS OF 5-CYANOPYRIMIDINES'

## Beschreibung

Die Erfindung betrifft ein Verfahren zur Herstellung von 4-Amino-2-chlor-5-cyan-6-(methylthio)pyrimidin (I) 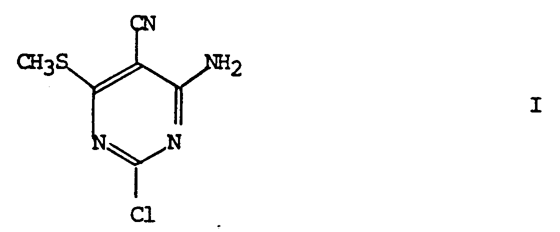
2-Chlorpyrimidine sind Zwischenprodukte zur Synthese von 2-Aminopyrimidinen, einer Substanzklasse, die zahlreiche wirksame Pestizide enthält.
Ein wichtiger Vertreter der 2-Chlorpyrimidine ist das 4-Amino-2-chlor-5-cyan-6-(methylthio)pyrimidin, dessen Methylthio-Gruppe, gegebenenfalls nach Oxidation zur Methansulfonyl-Gruppe, nucleophil ausgetauscht werden kann (EP-Anm. 0 244 360).
Das bekannte Verfahren zur Herstellung von 4-Amino-2-Chlor-5-cyan-6-(methylthio)pyrimidin (H.Kristinsson, J.Chem.Soc. Chem.Commun. 1974, 350) geht von Cyanamid und Schwefelkohlenstoff aus, die mit Kaliumhydroxid das Dikaliumsalz der Cyanimidodithiokohlensäure liefern (A.Hantzsch und M.Wolvekamp, Justus Liebigs Ann.Chem. 331, 282 (1904)). Dieses wird mit Dimethylsulfat zu Dimethylcyanimidodithiocarbonat umgesetzt, welches in Gegenwart von Natriumethylat Malonsäurenitril addiert. Bei Zusatz von Chlorwasserstoff cyclisiert das Additionsprodukt zum Pyrimidin. Dabei entsteht jedoch nicht nur das gewünschte Produkt, sondern auch das isomere 2-Amino-4-chlor-5-cyan-6-(methylthio)pyrimidin, und zwar im Verhältnis 3:2 (2-Amino-/4-Amino-), so dass bei einer Gesamtausbeute von 88% die nutzbare Ausbeute nur ca. 35% beträgt.

Aufgabe der vorliegenden Erfindung war daher, ein Verfahren bereitzustellen, das eine hohe Ausbeute an 4-Amino-2-chlor-5-cyan-6-(methylthio)pyrimidin und nur geringe Mengen an Nebenprodukten liefert.
Erfindungsgemäss wird diese Aufgabe durch das Verfahren nach Patentanspruch 1 gelöst.

Es wurde überraschend gefunden, dass 4-Amino-2-chlor-5-cyan-6-(methylthio)pyrimidin in sehr guter Ausbeute und praktisch frei von Nebenprodukten erhalten werden kann, indem zunächst das Malonsäurenitril mit Schwefelkohlenstoff und einem Alkalialkoholat zu dem entsprechenden Dialkalisalz der Dicyandithioessigsäure (II) 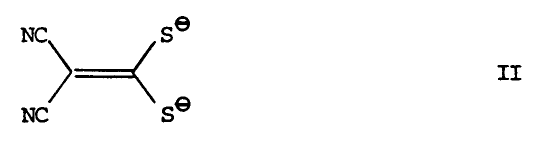
umgesetzt und anschliessend mit einem Methylierungsmittel, beispielsweise Dimethylsulfat, in das Dicyanketendimethylmercaptal (III) 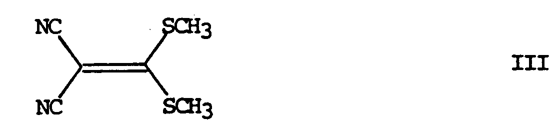
übergeführt wird. Letzteres wird mit Cyanamid in Gegenwart einer Base zu dem Anion des entsprechenden Dicyanketen-S,N-acetals (IV) 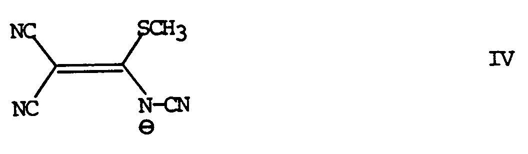
kondensiert, das analog zu dem bekannten Verfahren in Gegenwart von Chlorwasserstoff zu der Zielverbindung cyclisiert werden kann.

Der erste Teil der Synthese bis zum Dicyanketendimethylmercaptal ist an sich bekannt (R.Gompper und W.Töpfl, Chem.Ber. 95, 2861 (1962)). Die Literaturausbeute (68%) kann noch deutlich gesteigert werden, wenn zunächst aus dem vorgelegten Malonsäurenitril mit einem Aequivalent Base das Anion gebildet wird und der Schwefelkohlenstoff simultan mit dem zweiten Aequivalent Base zudosiert wird, statt Schwefelkohlenstoff und Base alternierend portionsweise zuzugeben.

Als Base für die Kondensation des Dimethylmercaptals mit Cyanamid wird vorzugsweise ein Alkalialkoholat verwendet. Besonders bevorzugt sind Natriumalkoholate, insbesondere Natriumethylat. Es ist jedoch auch möglich, andere Basen, wie beispielsweise Alkalihydroxide, einzusetzen.

Die Kondensation wird zweckmässig in einem polaren Lösungsmittel, beispielsweise in einem niederen Alkohol, durchgeführt.

Wenn als Base ein Alkoholat eingesetzt wird, wird als Lösungsmittel vorzugsweise der entsprechende Alkohol verwendet, also beispielsweise Ethanol bei Natriumethylat als Base.
Bei Verwendung von Hydroxiden oder schwächeren Basen kann auch Wasser oder ein wässriges Lösungsmittelgemisch eingesetzt werden.
Die Kondensation wird vorzugsweise etwa bei Umgebungstemperatur durchgeführt, so dass weder Heizung noch Kühlung erforderlich ist, also etwa im Bereich von 10 bis 40°C.

Das Reaktionsprodukt IV wird vorteilhaft nach Abdestillieren des Lösungsmittels ohne Reinigung mit Chlorwasserstoff versetzt und cyclisiert. Chlorwasserstoff wird vorzugsweise in Form der wässrigen Chlorwasserstoffsäure eingesetzt, besonders bevorzugt in einer Konzentration von 4 bis 8 M.

Auch die Cyclisierung kann im Bereich der Umgebungstemperatur ohne besondere Massnahmen zur Temperierung durchgeführt werden.

Die nachfolgenden Beispiele verdeutlichen die Durchführung des erfindungsgemässen Verfahrens.

### Beispiel 1

### Dicyanketendimethylmercaptal

Zu einer Natriumethylatlösung aus 2,3 g (0,1 mol) Natrium und 41 g Ethanol wurden unter Feuchtigkeitsausschluss innerhalb von 5 min bei Raumtemperatur unter Rühren 6,6 g (0,1 mol) Malonsäuredinitril (geschmolzen) zugetropft und noch weitere 5 min bei Raumtemperatur gerührt.
Die entstandene Suspension wurde auf 15°C abgekühlt, und bei dieser Temperatur wurden innerhalb von 60 min aus zwei synchron betätigten Injektionsspritzen die Lösungen von 7,6 g (0,1 mol) Schwefelkohlenstoff in 36 g Ethanol und 2,3 g (0,1 mol) Natrium in 41 g Ethanol zugegeben. Während der Zugabe bildete sich eine klare gelb-grüne Lösung, die noch weitere 60 min gerührt wurde.
Anschliessend wurden aus einem Tropftrichter innerhalb von 30 min unter Rühren 26,5 g (0,21 mol) Dimethylsulfat zugegeben. Die Temperatur stieg dabei an und wurde durch Kühlung auf 20°C gehalten.
Es entstand eine gelbe Suspension, die noch 4 h bei 20°C gerührt und anschliessend unter Rühren in 400 g Eiswasser gegossen wurde. Die wässrig-ethanolische Suspension wurde zur Zersetzung von überschüssigem Dimethylsulfat noch 2 h bei Raumtemperatur gerührt, auf 5°C abgekühlt und filtriert.
Der Filterkuchen wurde mit wenig Wasser gewaschen und bei Raumtemperatur im Vakuum getrocknet.
- Ausbeute:: 14,1 g gelbliche Kristalle, Gehalt (HPLC) 99,9% (83% d.Th., bezogen auf Malonsäuredinitril).
- Schmp.:: 78-79,5°C (Lit. 81°C)

### Beispiel 2

### 4-Amino-2-chlor-5-cyan-6-(methylthio)pyrimidin

Aus 0,23 g Natrium (10 mmol) und 25 ml Ethanol wurde eine Natriumethylatlösung hergestellt. Darin wurden 0,42 g Cyanamid (10 mmol) aufgelöst und anschliessend 1,70 g Dicyanketendimethylmercaptal (10 mmol) zugegeben. Die sich bildende gelbliche Suspension wurde 1 h bei 20°C gerührt und anschliessend zur Trockene eingeengt.
Zu dem Rückstand (1,98 g gelbes Pulver) wurde bei 0°C innerhalb von 15 min eine Mischung von 20 ml konz. Salzsäure und 12 ml Wasser gegeben.
Die entstandene gelbliche Suspension wurde noch 20 h bei Raumtemperatur gerührt. Das feste Produkt wurde abfiltriert, mit wenig Wasser gewaschen, in 60 ml 10%iger Natriumcarbonatlösung aufgeschlämmt, nochmals abfiltriert und mit Wasser gewaschen. Schliesslich wurde das Produkt bei 30°C/30 mbar getrocknet.
- Ausbeute:: 1,95 g gelbliches Pulver, Gehalt (GC) 96% (93% der Theorie)
- Schmp.:: ca. 268°C

## Patentansprüche

1. Verfahren zur Herstellung von 4-Amino-2-chlor-5-cyan-6-(methylthio)pyrimidin der Formel 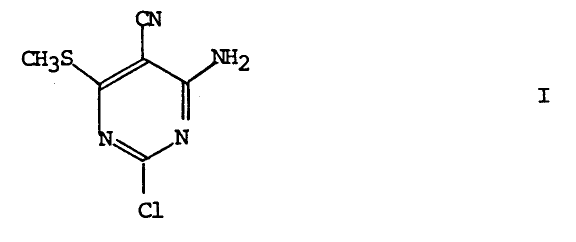 dadurch gekennzeichnet, dass in einer ersten Stufe Malonsäuredinitril mit Schwefelkohlenstoff in Gegenwart einer starken Base zum Dianion der Dicyandithioessigsäure der Formel 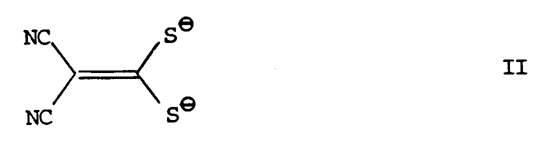 umgesetzt und anschliessend mit einem Methylierungsmittel zum Dicyanketendimethylmercaptal der Formel 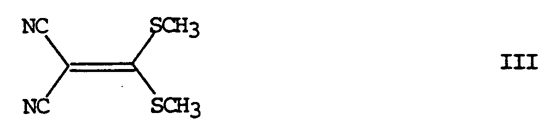 methyliert wird, welches mit Cyanamid in Gegenwart einer Base zum Anion des 2-Cyan-3-cyanamino-3-methylthio-acrylnitrils der Formel 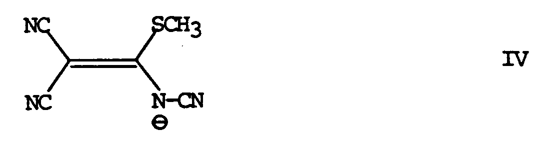 kondensiert und anschliessend in Gegenwart von Chlorwasserstoff zum 4-Amino-2-chlor-5-cyan-6-(methylthio)pyrimidin cyclisiert wird.

2. Verfahren gemäss Patentanspruch 1, dadurch gekennzeichnet, dass als Base jeweils ein Alkalialkoholat eingesetzt wird.

3. Verfahren gemäss Patentanspruch 2, dadurch gekennzeichnet, dass bei den Umsetzungen in Gegenwart von Alkalialkoholat jeweils der entsprechende Alkohol als Lösungsmittel verwendet wird.

4. Verfahren gemäss Patentanspruch 2 oder 3, dadurch gekennzeichnet, dass als Alkalialkoholat Natriumethylat eingesetzt wird.

5. Verfahren gemäss einem oder mehreren der Patentansprüche 1 bis 4, dadurch gekennzeichnet, dass bei der Umsetzung des Malonsäuredinitrils mit dem Schwefelkohlenstoff das Malonsäuredinitril mit einem Aequivalent Base vorgelegt und der Schwefelkohlenstoff synchron mit einem zweiten Aequivalent Base zugegeben wird.

6. Verfahren gemäss einem oder mehreren der Patentansprüche 1 bis 5, dadurch gekennzeichnet, dass als Methylierungsmittel Dimethylsulfat eingesetzt wird.

7. Verfahren gemäss einem oder mehreren der Patentansprüche 1 bis 6, dadurch gekennzeichnet, dass der Chlorwasserstoff als wässrige Chlorwasserstoffsäure eingesetzt wird.

## Claims

1. Process for the preparation of 4-amino-2-chloro-5-cyano-6-(methylthio)pyrimidine of formula 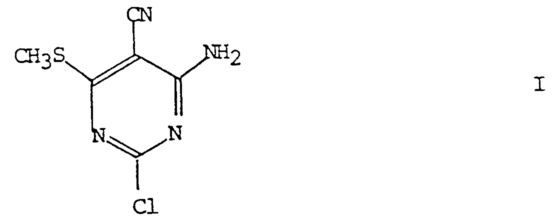 characterized in that in a first step malonic dinitrile is reacted with carbondisulfide in the presence of a strong base to the dianion of dicyanodithioacetic acid of formula 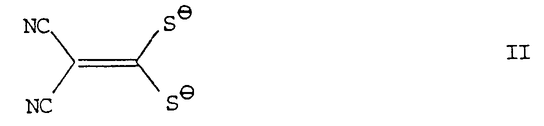 and is then methylated with a methylating agent to the dicyanoketene dimethylmercaptal of formula 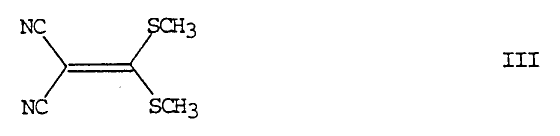 which latter is condensed with cyanamide in the presence of a base to the anion of 2-cyano-3-cyanamino-3-methylthio acrylonitrile of formula 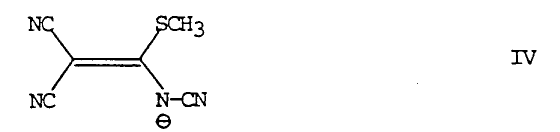 and thereafter cyclized to 4-amino-2-chloro-5-cyano-6-(methylthio)pyrimidine in the presence of hydrogen chloride.

2. Process according to claim 1, characterized in that an alkali alcoholate is used as the base.

3. Process according to claim 2, characterized in that the reactions in the presence of alkali alcoholate are carried out in the respective alcohol as the solvent.

4. Process according to claim 2 or 3, characterized in that sodium ethylate is used as the alkali alcoholate.

5. Process according to one or more of claims 1 to 4, characterized in that in the reaction of malonic dinitrile with carbondisulfide malonic dinitrile is introduced with one equivalent of base and carbondisulfide is added synchronously with a second equivalent of base.

6. Process according to one or more of claims 1 to 5, characterized in that dimethylsulfate is used as the methylating agent.

7. Process according to one or more of claims 1 to 6, characterized in that the hydrogen-chloride is used as an aqueous solution of hydrochloric acid.

## Revendications

1. Procédé pour la préparation de 4-amino-2-chloro-5-cyano-6-(méthylthio)pyrimidine de la formule 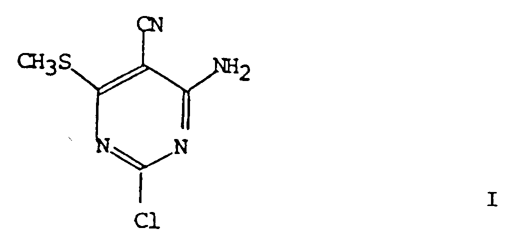 caractérisé en ce que dans une première étape, on transforme le dinitrile de l'acide malonique avec du sulfure de carbone, en présence d'une base forte en dianion de l'acide dicyandithioacétique de la formule 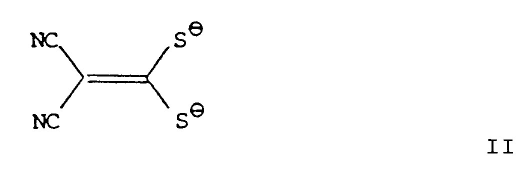 et consécutivement, on méthylise avec un agent de méthylisation en dicyancétènediméthylmercaptal de la formule 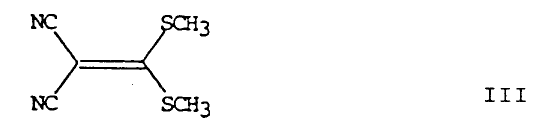 qui est condensé en présence d'une base en anion de 2-cyan-3-cyanamino-3-méthylthio-acrylnitriles de la formule 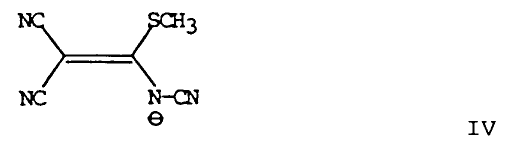 et consécutivement en présence de chlorure d'hydrogène, on cyclise en 4-amino-2-chloro-5-cyano-6-(méthylthio)pyrimidine.

2. Procédé selon la revendication 1, caractérisé en ce qu'en tant que base on met chaque fois en oeuvre un alcoolat alcalin.

3. Procédé selon la revendication 2, caractérisé en ce que pour les réactions en présence d'alcoolat alcalin, on utilise chaque fois l'alcool correspondant en tant que solvant.

4. Procédé selon la revendication 2 ou 3, caractérisé en ce qu'en tant qu'alcoolat alcalin, on utilise du méthylate de sodium.

5. Procédé selon une ou plusieurs des revendications 1 à 4, caractérisé en ce que pour la conversion du dinitrile de l'acide malonique avec le sulfure de carbone, on introduit le dinitrile de l'acide malonique avec un équivalent base et l'on ajoute le sulfure de carbone de façon synchronisée avec un deuxième équivalent base.

6. Procédé selon une ou plusieurs des revendications 1 à 5, caractérisé en ce qu'en tant qu'agent de méthylation, on met en oeuvre du sulfate de diméthyle.

7. Procédé selon une ou plusieurs des revendications 1 à 6, caractérisé en ce que le chlorure d'hydrogène est mis en oeuvre sous forme d'acide chlorhydrique aqueux.
